## Europäisches Patentamt

## European Patent Office

## Office européen des brevets

(19)

(11) Numéro de publication: **0 193 416**
**B1**

(12) # FASCICULE DE BREVET EUROPÉEN

(45) Date de publication du fascicule du brevet:
**14.12.88**

(51) Int. Cl.⁴: **A 01 N 59/00,** A 61 L 2/18

(21) Numéro de dépôt: **86400036.9**

(22) Date de dépôt: **09.01.86**

(54) **Composition aseptisante pour lentilles de contact.**

(30) Priorité: **30.01.85 FR 8501301**

(43) Date de publication de la demande:
**03.09.86 Bulletin 86/36**

(45) Mention de la délivrance du brevet:
**14.12.88 Bulletin 88/50**

(84) Etats contractants désignés:
**BE CH DE FR GB IT LI NL SE**

(56) Documents cité:
**DE-A-3 134 050**
**FR-A-2 247 327**
**GB-A-1 570 492**

(73) Titulaire: **L'AIR LIQUIDE, SOCIETE ANONYME
POUR L'ETUDE ET L'EXPLOITATION DES
PROCEDES GEORGES CLAUDE, 75, Quai d'Orsay,
F-75321 Paris Cédex 07 (FR)**

(72) Inventeur: **Le Rouzic, Daniel, 23, avenue des Lilas,
F-95120 Ermont (FR)**
Inventeur: **Jourdan- Laforte, Eric, 67, rue La
Fontaine, F-75016 Paris (FR)**

(74) Mandataire: **Bouton Neuvy, Liliane, L'Air liquide,
Société Anonyme pour L'Etude et L'Exploitation
des Procédés Georges Claude 75, Quai d'Orsay,
F-75321 Paris Cédex 07 (FR)**

LIBER, STOCKHOLM 1988

EP 0 193 416 B1

## Description

La présente invention concerne une composition aseptisante pour lentilles de contact.

L'utilisation de lentilles de contact implique un traitement d'aseptisation quotidien, et de nombreux et variés procédés de trampage et de stérilisation ont été proposés.

Certaines compositions commerciales avaient pour base des agents aseptisants de la classe des sels de chlorohexidine ou de mercurothiolate de sodium. Selon le brevet US-3 689 673, les lentilles de contact souples hydrophiles sont traitées par trempage dans une solution aqueuse contenant de 0,001 à 0,1 % de chlorohexidine pendant un temps suffisant, et une stérilisation est obtenue en deux à trois heures avec une solution de concentration 0,001 % et en trente minutes à une concentration de 0,01 %.

Ces produits aseptisants ne sont actuellement plus utilisés car ils présentent l'inconvénient de favoriser la création de dépôts sur les lentilles de contact.

Les lentilles de contact souples sont généralement fabriquées à partir de polymères hydrophiles. Or, les groupes hydroxy des lentilles attirent et retiennent de substantielles quantités d'eau dans le plastique, et ceci conduit à des inconvénients au cours du nettoyage et de la stérilisation. De plis, on rencontre des difficultés supplémentaires dans le traitement des lentilles de contact souples hydrophiles, en raison de l'aptitude de celles-ci à la complexation et concentration de certains conservateurs utilisés pour les lentilles conventionnelles, tels le chlorobutanol, le chlorure de benzolconium, le thimérosal, le nitrate de phénylmercure... qui sont généralement inactivés à l'état de complexe. Et, si les conservateurs concentrés sont relâchés trop rapidement sur la cornée, ils peuvent causer des brûlures chimiques. Par conséquent, les solutions de nettoyage actuellement disponibles pour les lentilles conventionnelles dures ne conviennent pas pour les lentilles de contact souples.

L'efficacité du peroxyde d'hydrogène en tant que désinfectant et son usage comme agent germicide dans la désinfection de multiples appareils et dispositifs à usages médical sont bien connus.

Dès 1972, le peroxyde d'hydrogène a été signalé comme agent intéressant dans la stérilisation des lentilles de contact, en page 247 de "Soft contact Lens", publié par C.V. Mosby Co. Saint Louis.

Et la plupart des produits employés actuellement sont à base de solution aqueuse de peroxyde d'hydrogène à une concentration de l'ordre de 3 %.

Toutefois, avant son introduction dans l'oeil, une lentille de contact souple, traitée par le peroxyde d'hydrogène, doit être rincée plusieurs fois dans une solution tamponnée isotonique à pH 6,9 - 7,1. Un rinçage correct, correspondant à des immersions et rinçages répétés, voire jusqu'à quatre, nécessite du temps, de l'ordre de trente minutes et plus, de grands volumes de solution saline et sans certitude de reproductibilité suivant le manipulateur. En outre, le rinçage dilue simplement le peroxyde d'hydrogène jusqu'à une concentration plus faible, or le peroxyde d'hydrogène même en quantités très minimes de l'ordre de la dizaine de p.p.m. (parties par million) peut provoquer une irritation de l'oeil.

En conséquence, il a été recherché des procédés d'élimination du peroxyde d'hydrogène des lentilles de contact souples. Le brevet français n° 2 231 395 propose un procédé de traitement d'une lentille de contact souple pour effectuer son nettoyage et sa stérilisation et la traiter pour son introduction dans l'oeil après une stérilisation complète, consistant à placer la lentille de contact souple dans une solution de stérilisation aqueuse à base de peroxyde d'hydrogène contenant une concentration approximativement isotonique de chlorure de sodium et une quantité catalytique d'un catalyseur de décomposition du peroxyde d'hydrogène et à maintenir la lentille de contact dans ladite solution jusqu'à ce que la concentration du peroxyde d'hydrogène présent soit inférieure à 10 p.p.m. environ. La lentille de contact souple est ainsi efficacement traitée et stérlisée par exposition à l'action du peroxyde d'hydrogène pendant une durée de dix minutes. Cependant, on laisse ensuite les lentilles de contact souples dans ce système aqueux de peroxyde d'hydrogène pendant environ six heures. En opérant dans ces conditions, les lentilles sont stérilisées et la quantité de peroxyde d'hydrogène résiduel décelable est de 20 p.p.m., pour réduire la quantité à moins de 10 p.p.m., un seul rinçage dans la solution saline isotonique suffit.

Le brevet européen n° 0 110 609 propose un traitement stérilisant par le peroxyde d'hydrogène associé à la neutralisation des quantités résiduaires de peroxyde d'hydrogène par une solution de pyruvate de sodium. Les lentilles sort mises au contact du peroxyde d'hydrogène pendant au moins environ 5 minutes, de préférence 10 minutes, elles sont ensuite immergées dans la solution de pyruvate de sodium, puis lavées avec une solution stérile aqueuse, isotonique.

Mais il a été constaté que le peroxyde d'hydrogène est peu efficace dans la stérilisation des lentilles de contact, notamment comme fongi ci de et certains dépôts sur les lentilles sont imputables à la présence de microorganismes.

Le brevet français 2 247 327 concerne le nettoyage et la rénovation d'objets en matière plastique notamment de lentilles de contact. Selon ce procédé de mise en oeuvre complexe, on fait alternativement subir à la lentille une dilatation et une contraction pour contribuer à l'élimination des résidus et salissures. La lentille oculaire est mise successivement au contact d'une première solution aqueuse acide contenant un peroxocomposé, puis d'une seconde solution aqueuse basique contenant un peroxocomposé, puis la lentille est mise au contact d'un détergent non-ionique, et soumise à un rinçage avec de l'eau. La première et deuxième solutions contiennent 0,1 à 15 % en poids d'un peroxocomposé utilisé pour son grand pouvoir de nettoyage et de blanchiment.

Il a été trouvé une composition aseptisante pour lentilles de contact, de type conventionnel dures ou de lentilles souples, ayant un pouvoir antiseptique bien supérieur à celui du peroxyde d'hydrogène, notamment comme fongicide, et ayant un effet moins irritant pour l'oeil que les solutions de peroxyde d'hydrogène, compatible avec les polymères utilisés pour réaliser les lentilles souples et permettant d'éviter la génération de dépôts sur les lentilles.

Cette composition stable, prête à l'emploi, à base de peroxyde d'hydrogène contient de l'acide peracétique, CH3COOOH, à faible concentration. On obtient des résultats très satisfaisants, avec une composition aseptisante contenant de 0,005 à 0,1 % en poids d'acide peracétique, 1 à 8 % en poids de peroxyde d'hydrogène et la quantité d'acide acétique nécessaire pour atteindre l'équilibre du système. Des teneurs pondérales de 0,01 à 0,04 %, en particulier de 0,01 à 0,015 % en poids sont particulièrement adaptées. On peut citer une solution contenant environ 3 % en poids de peroxyde d'hydrogène, environ 0,01 % en poids d'acide peracétique et la quantité d'acide acétique pour l'équilibre du système.

La composition aseptisante contient, en outre, un tensioactif non ionique présent à raison de 0,001 à 0,005 % en poids. Les tensio-actifs de la classe des polyéthanoxyalkyéther, condensats d'oxyéthylène et d'alcools gras promaires synthétiques, totalement biodégradables, conviennent parfaitement à l'application.

De plus, la composition contient de l'eau désionisée en quantité suffisante pour compléter à 100 %.

On peut obtenir la composition aseptisante, stable, prête à l'emploi par différentes méthodes, soit en recourant à la préparation de l'acide peracétique à partir de l'acide acétique et du peroxyde d'hydrogène, dans des proportions correspondant à la teneur attendue en acide peracétique, puis en procédant à l'addition du tensio-actif, à la complémentation de la solution par de l'eau désionisée, et ensuite au mûrissement jusqu'à l'équilibre du système.

Il est aussi possible de préparer la composition aseptisante immédiatement prête à l'emploi à partir d'une solution commerciale stable à faible concentration en acide peracétique à laquelle on ajoute les autres constituants de la composition, à savoir le peroxyde d'hydrogène, l'acide acétique, le tensio-actif et la complémentation par de l'eau désionisée.

Les compositions aseptisantes selon l'invention donnent d'excellents résultats dans le traitement quotidien de nettoyage et de stérilisation des lentilles de contact, notamment des lentilles souples.

Selon ce procédé très simple et rapide, on place la lentille dans le porte-lentilles, on la met au contact de la solution aseptisante pendant une durée de l'ordre de quelques minutes, de préférence 2 à 5 minutes, 2 minutes étant grandement suffisantes, puis on soumet la lentille à un bref et simple rinçage à l'eau courante, pendant une durée de l'ordre de 30 secondes, et la lentille de contact efficacenent aseptisée est immédiatement prête pour son application sur l'oeil.

L'analyse polarographique à courant échantillonné en milieu sulfate de lithium 0,1 molaire indique que la concentration en acide peracétique résiduaire dans la lentille après un trempage de 10 minutes dans une solution à 0,013 % en acide peracftique et rinçage 30 secondes est inférieure à 4 ± 1 p.p.m. et six heures plus tard la même détermination indique 2 ± 1 p.p.m. (partie par million) dans un volume de 1,2 ml, sachant que la phase aqueuse pèse 1,2 mg dans la lentille, ce qui correspond à une quantité infime d'acide peracétique résiduaire.

Les compositions selon l'invention, en particulier la solution finale de l'exemple 1 à 0,013 % d'acide peracétique, ont été étudiées quant à leur pouvoir aseptique, l'effet irritant pour l'oeil, la compatibilité avec les lentilles souples hydrophiles et les dépôts sur les lentilles.

## Pouvoir antiseptique

La solution a été testée selon la norme AFNOR T 72 151 sur trois souches bactériennes:

- Pseudomonas aeruginosa    CMCM - 2 - 22
- Streptococcus faecalis    ATCC - 10541
- Klebsiella Pneumoniae    ATCC - 10031

Les résultats montrent que la solution réduit la population de ces bactéries par un facteur $10^5$ à 20°C alors que $H_2O_2$ à 3 % n'a pas d'activité bactéricide selon le protocole d'essai (réduction de $10^5$ après 5 minutes de contact).

En ce qui concerne le pouvoir fongicide, la solution a été testée selon la norme AFNOR NFT 72 200 sur le Candida albicans APCC-2091; pour une réduction de la population de $10^7$ à $10^2$ germes/ml, il faut moins de 5 minutes de temps de contact avec la solution de l'exemple 1 et plus de 45 minutes de temps de contact avec une solution d'$H_2O_2$ à 3 %.

La solution de l'exemple 1 diluée de moitié, c'est-à-dire contenant 65 ppm d'acide peracétique, permet d'obtenir une réduction par un facteur $10^5$ en 10 minutes de temps de contact alors qu'il faut un temps de contact supéreure à 120 minutes pour obtenir le même résultat avec une solution aqueuse d'$H_2O_2$ à 1,5 %.

Sur l'Aspergillus niger 218 IP, le même protocole appliqué à une population de $4 \times 10^6$ spores/ml exige 20 minutes de temps de contact pour abaisser la population à $4 \times 10^1$ avec la solution de exemple 1 et 50 minutes avec une solution aqueuse d'$H_2O_2$ à 3 %.

3

**Effet irritant pour l'oeil**

Le test d'évaluation de l'irritation sur l'oeil a été effectué selon la méthode décrite par l'arrêté passé au Journal Officiel Français du 24 octobre 1984; selon le principe d'une application pendant 3 minutes sur l'oeil du lapin, d'une lentille traitée avec le produit antiseptique et non rincée.

On note les effet provoqués après 6 heures, 24 heures, et entuellement chaque jour pendant sept jours.

Selon ce test, les produits antiseptiques se situent de la manière suivante:

- solution aqueuse à 3 % d'$H_2O_2$: légèrement irritante.
- solution de l'exemple 1: très faiblement irritante.

La solution de l'exemple 1 se situe dans la même classe qu'une solution aqueuse à 0,9 % de chlorure de sodium.

Il est surprenant de constater que la solution de l'exemple 1 a un effet irritant sur l'oeil très peu prononcé malgré la présence d'acide peracétique et d'acide acétique, et imprévisible que cet effet soit inférieur a celui du peroxyde d'hydrogène à 3 % et que ladite composition soit non nécrosante.

**Compatibilité avec les lentilles souples hydrophiles**

Les tests ont été effectués sur des lentilles souples hydrophiles en polyhydroxyméthacrylate; les lentilles ont été trempées pendant 16 jours dans la solution de l'exemple 1, renouvelée quotidinnement avec un témoin conservé dans une solution isotonique à 0,9 % de chlorure de sodium.

Après environ 400 heures de traitement, on ne détecte pas de modification significative de la taille des lentilles, de leur résistance mécanique, de leur indice de réfraction et de leur élasticité; aucun effet irréversible n'a été observé, notamment le taux d'hydratation des lentilles dont dépend l'indice de réfraction.

**Dépôts sur les lentilles**

Sur ce point, les résultats sont aussi pleinement satisfaisants car il n'a pas été constaté de dépôts sur les lentilles utilisées et traitées avec la solution de l'exemple 1;

Il est donné, ci-après, des exemples qui illustrent l'invention à titre non limitatif.

**Exemple 1**

Dans un bécher d'un litre, on pèse 5 g d'acide acétique purifié à 99,7 %, on ajoute 41 g d'une solution commerciale de peroxyde d'hydrogène a 70 % et 500 g d'eau bipermutée distillée; la solution ainsi obtenue est agitée au moyen d'un agitateur magnétique pendant environ 5 minutes pour homogénéiserle mélange. On ajoute à la solution 200 mg d'alcool décylique éthoxylé et on complète le bain à 1000 g de solution avec de l'eau bipermutée distillée; et l'on poursuit l'agitation pendant 5 à 10 minutes. Puis on laisse mûrir la solution obtenue, à l'abri des poussières, jusqu'à l'équilibre du système.

La composition ainsi préparée la formule suivante exprimée en pourcentages pondéraux:

| | |
|---|---|
| acide péracétique | 0,013 % |
| acide acétique | 0,487 % |
| $H_2O_2$ | 2,850 % |
| alcool décylique éthosxylé | 0,002 % |
| (marque commerciale "Empilan KA 5") | |
| eau désionisée | 96,648 % |

Après huit mois de stockage à l'abri des poussières et de la lumière à une température de 20 ± 5°C, le titre en acide peracétique n'a pratiquement pas varié.

**Exemple 2**

Dans un bécher d'un litre, on pèse 15 g d'acide acétique purifié à 99,7 %, on ajoute 42 g d'une solution commerciale de peroxyde d'hydrogène à 70 % et 500 g d'eau bipermutée distillée; la solution ainsi obtenue est agitée au moyen d'un agitateur magnétique pendant environ 5 minutes pour homogénéiser le mélange. On ajoute à la solution 20 mg d'alcool décylique éthoxylé et on complète le bain à 1000 g de solution avec de l'eau bipermutée distillée; et l'on poursuit l'agitation pendant 5 à 10 minutes, puis on laisse mûrir la solution comme précédemment, et à l'équilibre sa composition pondérale est la suivante:

| | |
|---|---|
| acide peracétique | 0,043 % |
| acide acétique | 1,464 % |
| $H_2O_2$ | 2,920 % |
| alcool décylique éthoxylé | 0,005 % |
| eau désionisée | 95,568 % |

Après huit mois de stockage à l'abri des poussières et de la lumière à une température de 20 ± 5°C, le titre en acide peracétique n'a pratiquement pas varié.

## Exemple 3

On introduit dans 500 g d'eau bipermutée distillée, 5 g d'une solution commerciale à 2,5 % d'acide peracétique, préparée selon le brevet européen n° 0 024 219, vendue sous la marque commerciale "Bactipal", 40 g d'une solution commerciale de peroxyde d'hydrogène à 70 %, 4,6 g d'acide acétique, on homogénéise le mélange, puis on ajoute 20 mg d'alcool décylique éthoxylé, on complète à 1000 g de solution avec de l'eau bipermutée distillée, et poursuit l'agitation pendant 5 à 10 minutes.

On obtient une solution de composition pondérale:

| | |
|---|---|
| acide peracétique | 0,012 % |
| acide acétique | 0,448 % |
| $H_2O_2$ | 2,855 % |
| alcool décylique éthoxylé | 0,002 % |
| eau désionisée | 96,643 % |

immédiatement prête à l'emploi.

## Revendications

1. Composition aseptisante pour lentilles de contact constituée par une solution aqueuse stable, prête à l'emploi à base de peroxyde d'hydrogène, caractérisée en ce qu'elle contient 0,005 à 0,1 % en poids d'acide peracétique, 1 à 8 % en poids de peroxyde d'hydrogène et la quantité d'acide acétique nécessaire pour atteindre l'équilibre du système.

2. Composition aseptisante pour lentilles de contact selon la revendication 1, caractérisée en ce qu'elle contient 0,01 à 0,04 % en poids d'acide peracétique.

3. Composition aseptisante pour lentilles de contact selon la revendication 1, caractérisée en ce qu'elle contient de 0,01 à 0,015 % en poids d'acide peracétique.

4. Composition aseptisante pour lentilles de contact selon la revendication 1, caractérisée en ce qu'elle contient environ 3 % en poids de peroxyde d'hydrogène, environ 0,01 % en poids d'acide peracétique et la quantité d'acide acétique nécessaire pour maintenir l'équilibre du système.

5. Composition aseptisante pour lentilles de contact selon la revendication 1, caractérisée en ce que la composition contient, en outre, un tensio-actif non ionique présent à raison de 0,001 à 0,005 % en poids.

6. Composition aseptisante pour lentilles de contact selon la revendication 5, caractérisée en ce que le tensio-actif non ionique est un polyéthanoxyalkyléther.

7. Application de la composition aseptisante selon l'une quelconque des revendications 1 à 6, au traitement quotidien de nettoyage et stérilisation des lentilles de contact en particulier des lentilles de contact souples.

8. Procédé de traitement d'une lentille de contact en vue de son nettoyage et de sa stérilisation complète, caractérisé en ce qu'on place la lentille au contact de la solution aseptisante selon une quelconque des revendications 1 à 6, pendant une durée de l'ordre de quelques minutes, de préférence 2 à 5 minutes, puis la soumet à un bref et simple rinçage à l'eau courante, pendant une durée de l'ordre de 30 secondes.

## Patentansprüche

1. Desinfizierende Zusammensetzung für Kontaktlinsen, bestehend aus einer verwendungsbereiten stabilen wäßrigen Lösung auf der Basis von Wasserstoffperoxid, dadurch gekennzeichnet, daß sie 0,005 bis 0,1 Gew.-% Peressigsäure, 1 bis 8 Gew.-% Wasserstoffperoxid und die für ein Erreichen des Gleichgewichts des Systems erforderliche Menge an Essigsäure enthält.

2. Desinfizierende Zusammensetzung für Kontaktlinsen nach Anspruch 1, dadurch gekennzeichnet, daß sie 0,01 bis 0,04 Gew.-% Peressigsäure enthält.

3. Desinfizierende Zusammensetzung für Kontaktlinsen nach Anspruch 1, dadurch gekennzeichnet, daß sie 0,01 bis 0,015 Gew.-% Peressigsäure enthält.

4. Desinfizierende Zusammensetzung für Kontaktlinsen nach Anspruch 1, dadurch gekennzeichnet, daß sie etwa 3 Gew.-% Wasserstoffperoxid, etwa 0,01 Gew.-% Peressigsäure und die für eine Aufrechterhaltung des Gleichgewichts des Systems erforderliche Menge an Essigsäure enthält.

5. Desinfizierende Zusammensetzung für Kontaktlinsen nach Anspruch 1, dadurch gekennzeichnet, daß die Zusammensetzung außerdem ein nicht-ionisches Tensid in einer Menge von 0,001 bis 0,005 Gew.-% enthält.

6. Desinfizierende Zusammensetzung für Kontaktlinsen nach Anspruch 5, dadurch gekennzeichnet, daß das nicht-ionische Tensid ein Polyethanoxyalkylether ist.

7. Verwendung der desinfizierenden Zusammensetzung nach einem der Ansprüche 1 bis 6 zur täglichen Reinigungs- und Sterilisationsbehandlung von Kontaktlinsen, insbesondere von reichen Kontaktlinsen.

8. Verfahren zur Behandlung einer Kontaktlinse im Hinblick auf eine Reinigung und vollständige Sterilisation derselben, dadurch gekennzeichnet, daß man die Linse während einer Dauer in der Größenordnung von einigen Minuten, vorzugsweise von 2 bis 5 min, in Berührung mit der desinfizierenden Lösung nach einem der Ansprüche 1 bis 6 bringt und sie dann einem kurzen und einfachen Spülen mit fließendem Wasser während einer Dauer in der Größenordnung von 30 sec unterzieht.


## Claims

1. Disinfecting composition for contact lenses constituted by a stable aqueous solution ready for use based on hydrogen peroxide, characterised in that it contains 0,005 to 0,1 % by weight of peracetic acid, 1 to 8 % by weight of hydrogen peroxide and the quantity of acetic acid necessary to attain equilibrium in the system.

2. Disinfecting composition for contact lenses according to claim 1, characterised in that it contains 0,01 to 0,04 % by weight of peracetic acid.

3. Disinfecting composition for contact lenses according to claim 1, characterised in that it contains 0,01 to 0,015 % by weight of peracetic acid.

4. Disinfecting composition for contact lenses according to claim 1, characterised in that it contains about 3 % by weight of hydrogen peroxide, about 0,01 % by weight of peracetic acid and the quantity of acetic acid necessary to maintain the equilibrium of the system.

5. Disinfecting composition for contact lenses according to clam 1, characterised in that the composition contains in addition from 0,001 to 0,005% by weight of a non-ionic surfactant.

6. Disinfecting composition for contact lenses according to claim 5, characterised in that the non-ionic surfactant is a polyethanoxyalkylether.

7. Application of the disinfecting composition according to any one of claims 1 to 6 in the daily cleaning and sterilisation treatment of contact lenses, in particular flexible contact lenses.

8. Process for the treatment of a contact lens by its cleaning and complete sterilisation, characterised in that the lens is placed in contact with the disinfecting solution according to any one of claims 1 to 6 for a period of the order of several minutes, preferably 2 to 5 minutes, then it is submitted to the brief and simple rinsing in running water for a period of the order of 30 seconds.